# DEMANDE DE BREVET EUROPEEN

(11) **EP 3 738 579 A1**
(43) Date de publication de la demande: **18.11.2020**
(21) Numéro de dépôt: 20173400.1
(22) Date de dépôt: 07.05.2020
(51) Int. Cl.: A61K 8/9789, A61K 36/61, A61P 17/16, A61Q 19/00, A61Q 19/08

(54) **UTILISATION COSMETIQUE D'EXTRAIT D'EUGENIA JAMBOS POUR UN EFFET PROTECTEUR, NOTAMMENT ANTI-AGE, DE LA PEAU, DE SES ANNEXES ET DES MUQUEUSES**

(30) Priorité: 16.05.2019 FR 1905137
(71) Demandeur: Laboratoires De Biologie Vegetale Yves Rocher, 56200 La Gacilly (FR)
(72) Inventeur: BOUVART-GRIENENBERGER, Ingrid, 51100 REIMS (FR); LAUGIER-CASSIN, Florence, 92500 RUEIL-MALMAISON (FR); LAPERDRIX, Céline, 78470 SAINT-REMY-LES-CHEVREUSES (FR)
(74) Mandataire: Novagraaf Technologies

(57) **Abrégé**

L'invention se rapporte à l'utilisation cosmétique d'un extrait d'Eugenia jambos L. pour une action anti-âge de la peau et/ou de ses annexes et/ou des muqueuses.

La présente invention se rapporte également à une composition cosmétique ou dermatologique comprenant au moins un extrait d'Eugenia jambos L. et un véhicule cosmétiquement et/ou dermatologiquement acceptable, ladite composition comprenant de 0,00001 à 5 % en poids dudit extrait par rapport au poids total de la composition.

La présente invention se rapporte en outre à un dispositif se présentant sous une forme choisie parmi un pot, un flacon-pompe, une lingette, un masque, un dispositif transdermique, un patch et un spray, ledit dispositif comprenant la composition de l'invention, ainsi qu'à un procédé cosmétique non-thérapeutique comprenant l'application sur la peau ou les muqueuses de la composition cosmétique de l'invention.

## Description

### Domaine technique

La présente invention se rapporte à l'utilisation cosmétique d'un extrait d'Eugenia jambos L., ainsi qu'à une composition cosmétique ou dermatologique comprenant un extrait d'Eugenia jambos L., à un procédé cosmétique et à un dispositif mettant en œuvre la composition.

La présente invention trouve ses applications dans le domaine de la cosmétique et de la dermatologie, plus particulièrement de la cosmétique cutanée.

Dans la description ci-dessous, les références entre crochets ([ ]) renvoient à la liste des références présentée à la fin du texte.

### Etat de la technique

La peau est un organe vital à part entière qui se compose de trois tissus distincts, assumant chacun différents rôles grâce à différents types cellulaires et différentes structures.

Le tissu le plus en surface et donc le plus exposé est l'épiderme. Cet épithélium pluristratifié (Malpighien) et kératinisé, se compose de différentes cellules associées à de nombreuses fonctions de barrière et de protection. Les cellules majoritaires sont les kératinocytes qui se divisent dans la couche basale et entament leur différenciation jusqu'à la couche cornée (couche la plus externe) puis sont éliminés par desquamation, en 21 à 28 jours, en moyenne. L'épiderme comprend également des mélanocytes, cellules responsables de la pigmentation de la peau. Le rôle majeur de l'épiderme est d'apporter à la peau, et donc au corps humain, une première ligne de protection contre les agressions extérieures, comme les agressions physiques, chimiques, hydriques et bactériologiques. Cette protection est notamment assurée par les couches les plus différenciées et la couche cornée connue pour ses propriétés hydrophobes, son aspect compact et étanche. Une bonne différenciation des kératinocytes et donc une bonne desquamation sont essentielles pour une surface lisse, homogène et régulière. L'ensemble de ces mécanismes dépend directement des capacités prolifératives des kératinocytes. Or, les stress extérieurs et l'âge ralentissent ce processus qui peut alors devenir plus long et plus irrégulier. La desquamation devenant irrégulière et/ou anarchique, des défauts de lissage et d'homogénéité de surface de la peau s'installent.

En position intermédiaire, le derme est un tissu conjonctif investi majoritairement de fibroblastes et de protéines matricielles donnant à la peau ses qualités de compressibilité et d'élasticité connues. Les modifications de sa texture et de sa composition sont largement responsables des altérations de la peau qui surviennent au cours du vieillissement. Le relief de la couche cornée est, par ailleurs, directement conditionné par la qualité et la densité du derme qui la sous-tend.

Au sein de la trame conjonctive, s'intercalent aussi d'autres cellules et structures, tel un important réseau circulatoire et nutritif, constitué des vaisseaux sanguins et des capillaires lymphatiques, ainsi que les annexes épidermiques : cheveux, poils, ongles, glandes pilosébacées et glandes sudoripares, qui prennent naissance dans le derme profond.

L'hypoderme, situé en profondeur et constitué en majeure partie de lobules graisseux (adipocytes), assure une fonction de support primaire, de protection mécanique et thermique et joue aussi un rôle de stockage des réserves énergétiques rapidement mobilisables pour tous les besoins biologiques, comme par exemple le renouvellement cellulaire, la défense de l'organisme ou la contraction musculaire.

La peau est donc un organe externe qui, à ce titre, est la première ligne de défense de l'organisme vis-à-vis de l'environnement extérieur. Lequel, par ses variations rapides ou ses extrêmes, peut directement influencer la biologie et l'aspect esthétique de la peau, et provoquer des dégâts tissulaires plus ou moins réversibles et/ou un vieillissement prématuré. En effet, le vieillissement cutané n'est pas seulement dû à des facteurs intrinsèques, il est également fortement influencé par les facteurs environnementaux auxquels il est impossible à la peau de se soustraire et qui provoquent un stress cellulaire.

Il existe donc un réel besoin de renforcer la fonction barrière de la peau, et de lui permettre de se protéger efficacement contre tous les facteurs environnementaux de quelque nature qu'ils soient (pas uniquement solaire) pour limiter les stress occasionnés. Il existe aussi un réel besoin de soins cosmétiques pour lutter contre les effets du vieillissement de façon préventive, la plupart des soins existants comblant les effets du vieillissement de façon curative.

Par ailleurs, les attentes des consommateurs en termes de soins cosmétiques s'orientent de plus en plus vers des produits naturels, efficaces et éco-conçus. Il reste donc un réel besoin de trouver de nouvelles compositions et/ou composés naturels et éco-conçus permettant d'assurer de façon effective et complète un effet de protection de la peau vis-à-vis des agressions extérieures pour un bénéfice anti-âge.

En particulier, il existe un réel besoin de trouver des composés naturels qui permettent une protection cutanée efficace contre les stress hydriques, thermiques, physiques, UV, chimiques, visant à obtenir in fine des efficacités cosmétiques plus larges que les produits existants.

### Description de l'invention

La présente invention a précisément pour but de répondre à ces besoins et inconvénients de l'art antérieur.

Les inventeurs sont les tout premiers à utiliser un extrait d'Eugenia jambos L. permettant précisément de répondre efficacement aux besoins précités.

Les inventeurs sont les tous premiers à avoir découvert que cet extrait d'Eugenia jambos L. possède de manière inattendue un effet anti-âge sur la peau, en limitant les stress environnementaux et surtout leurs effets.

En effet, les inventeurs ont notamment mis en évidence un effet de cet extrait sur la diminution de la réponse aux stress environnementaux notamment UV et sur la fonction barrière naturelle de la peau.

Plus précisément, ils ont démontré un effet limitant l'expression des médiateurs cellulaires de stress et/ou d'inflammation, causant des dégradations au niveau cellulaire et tissulaire et un effet stimulant de la maturation cellulaire naturelle conduisant à l'édification de la fonction barrière épidermique à la surface de la peau.

Ainsi, les inventeurs sont les premiers à proposer cet extrait en tant qu'agent cosmétique anti-âge pour protéger et/ou préserver l'aspect esthétique de la peau, pour protéger et/ou préserver ses qualités biomécaniques, pour protéger et/ou préserver et/ou renforcer sa fonction barrière naturelle. Avantageusement, cet extrait permet également d'améliorer l'aspect de la peau, notamment il apporte un effet de lissage, et/ou de douceur et/ou d'homogénéisation de l'état de surface.

Ainsi, un premier objet de l'invention se rapporte à l'utilisation cosmétique d'un extrait d'Eugenia jambos L. pour une action anti-âge de la peau et/ou de ses annexes et/ou des muqueuses.

Eugenia jambos L. ou Syzygium jambos L. Asto (Myrtaceae), également appelée jamrosat et Pomme rose (pommerose) ou jambosier en Français, est un arbuste originaire du sud-est de l'Asie, probablement plus précisément de Malaisie. Il a été introduit par la suite en Inde, notamment dans l'état du Kerala. Aujourd'hui on peut trouver Eugenia jambos en Afrique, île Maurice, Réunion, Seychelles, Mexique, Etats Unis, Equateur, île Galapagos, Amérique du sud. Eugenia jambos est un arbre très ramifié (broussailleux) à feuilles persistantes pouvant atteindre 15m de haut. Ses feuilles sont lancéolées à l'apex acuminé (pointu) et à la base cunéiforme (en forme de cœur), plus ou moins coriaces. Son inflorescence à corymbe terminal est composée de 5 à 6 fleurs aux pétales blanchâtres qui se distinguent par ses nombreux filaments (étamines) jaune, très visibles et long (4cm environ). Mais ce sont ses fruits pyriformes surtout qui sont réputés : leur chair comestible a un parfum de rose. Réputé pour son utilisation en ornementation, il est également utilisé en alimentaire car les fruits sont mangés crus ou cuits dans des recettes régionales diverses. Cette plante est aussi utilisée en médecine traditionnelle. De nombreuses décoctions et préparations sont encore utilisées aujourd'hui en Asie pour traiter l'eczéma, la malaria, les troubles respiratoires et les maladies infectieuses. Plusieurs parties de l'arbre sont utilisées dans la médecine traditionnelle mondiale étant donné que l'espèce est largement répartie.

L'arbre est riche en tanins et pourrait avoir un intérêt antimicrobien. Le bois infusé aurait des propriétés antimicrobiennes (Corine Djadjo Djipa et al. : "Antimicrobial activity of bark extracts of Syzygium jambos". J Ethnopharmacol. 2000 Jul;71(1-2):307-13 ([1])).

Le fruit peut être consommé cru ou servir à des préparations de liqueurs parfumées. Riche en vitamine C, le fruit peut être mangé brut (cru) ou utilisé dans des recettes régionales diverses.

En médecine traditionnelle, les graines sont reconnues pour le traitement des diarrhées, ainsi que dans des préparations homéopathiques contre l'acné, la nausée, les céphalées, les brûlures d'estomac, les coliques, les diarrhées et la fièvre (P. Padma Rao : « Standardisation of Homoeopathic drug - Syzygium Jambos (L.) Alston ». New Delhi: Indian Journal of Research in Homoeopathy Vol. 5, No. 3, July - September, 2011 ([2])). Les parties aériennes (feuilles, tiges) sont utilisées en décoction en tant que traitement du diabète (Gavillán-Suárez et al. : « Chemical profile and in vivo hypoglycemic ». BMC Complement Altern Med. 2015 Jul 22;15:244 ([3])) et comme tonique pour le cerveau et le foie en Inde. Les fleurs sont utilisées en Inde pour réduire la fièvre. Les racines sont utilisées à Cuba comme traitement de l'épilepsie.

Les feuilles sont largement utilisées en médecine traditionnelle comme digestif/diurétique, anti-inflammatoire (K. Slowing, E. C. (1992). Anti-inflammatory activity of leaf extracts of Eugenia jambos. J Ethnopharmacol. 1994 Jun;43(1):9-11 ([4])) analgésique, fébrifuge, expectorant (Avila-Peña D. et al.: " Antinociceptive activity of Syzygium jambos leaves extract on rats". J Ethnopharmacol. 2007 Jun 13;112(2):380-5 ([5])), astringent, traitement des rhumatismes, et traitement des plaies oculaires.

On entend par « action anti-âge », au sens de la présente invention une action de prévention et/ou de retard et/ou de limitation des signes du vieillissement cutané, notamment du vieillissement chronologique (endogène) mais aussi du vieillissement accéléré (exogène) provoqué par les stress environnementaux, par exemple les ultraviolets, les variations et/ou les conditions extrêmes de température, d'hygrométrie, de pression, la pollution urbaine, domestique ou industrielle, la qualité de l'eau et/ou la qualité/composition des produits cosmétiques. L'action anti-âge peut passer par exemple par une action limitant préventivement ou curativement les effets néfastes cellulaires et/ou tissulaires, comme le ralentissement du métabolisme, la diminution de la synthèse des éléments de la matrice extra cellulaire, et/ou l'oxydation des structures moléculaires, provoqués par toutes les sources possibles du vieillissement cutané et conduisant à une altération de l'aspect esthétique de la peau. Ainsi l'action anti-âge peut être au moins une action choisie parmi l'action de protéger, réparer la peau, préserver l'aspect, améliorer l'aspect, préserver les qualités biomécaniques, préserver et/ou renforcer la fonction barrière naturelle, de la peau et/ou de ses annexes et/ou des muqueuses. Il peut s'agir notamment d'un effet de lissage, et/ou d'un effet d'amélioration de la douceur et/ou de l'homogénéité et/ou de l'éclat de la surface de la peau.

On entend par « qualités biomécaniques », au sens de la présente invention, les propriétés physiques qui sont propres à la peau, et notamment au derme. La peau est dotée d'une certaine plasticité qui lui permet de se déformer, d'être étirée, déplacée et de revenir en place (retour à la normale). Ces qualités biomécaniques et leurs amplitudes diminuent avec l'âge.

On entend par « amélioration de la fonction barrière de la peau », au sens de la présente invention, tout effet permettant d'améliorer la fonction barrière protectrice de l'épiderme. Il peut s'agir d'une action de stimulation de la différenciation des cellules épidermiques, conduisant à la transformation de kératinocytes proliférants de la couche basale à ceux de la couche granuleuse jusqu'à la couche cornée parfaitement étanches et organisés de façon à ne rien laisser passer, molécules ou agents exogènes. Il peut s'agir alternativement ou en complément d'une limitation de réactivité en cas d'agression externe, comme lors d'une action anti-inflammatoire.

On entend par « améliorer l'aspect de la peau », au sens de la présente invention, tout effet permettant d'obtenir un avantage esthétique sur l'aspect de la peau, suite à l'utilisation de l'extrait selon l'invention, par rapport à l'aspect de la peau avant utilisation de l'extrait selon l'invention.

L'utilisation cosmétique de l'invention peut avantageusement permettre d'améliorer les qualités de surface, donc esthétiques, de l'épiderme.

On entend par « lisser la surface de la peau », au sens de la présente invention, la diminution qualitative et quantitative des microreliefs de la peau, notamment de leur profondeur. Il peut s'agir par exemple d'une diminution de la profondeur des rides et/ou des ridules.

On entend par « adoucir la peau », au sens de la présente invention, l'obtention d'un effet plus soyeux de la peau au toucher après utilisation de l'extrait selon l'invention, et/ou une diminution de l'effet de rugosité de la peau au toucher.

On entend par « améliorer l'éclat de la peau », au sens de la présente invention, l'obtention d'une peau plus lumineuse. Cet effet peut être observé qualitativement, par exemple visuellement, ou quantitativement, par exemple par des études de chromamétrie où les composantes L^{∗}a^{∗}b^{∗} sont mesurées.

Alternativement ou en complément, l'action anti-âge peut être une action de restauration, de maintien, de préservation et/ou de renforcement de l'hydratation de la peau, de ses annexes ou des muqueuses, cet effet étant distinct et complémentaire des effets précédemment cités.

On entend par « annexes », au sens de la présente invention, les annexes épidermiques qui prennent naissance dans le derme profond, comprenant au moins un élément choisi parmi les poils, les ongles, les glandes pilosébacées et les glandes sudoripares.

On entend par « muqueuse », au sens de la présente invention, notamment les muqueuses externes comme les lèvres.

On entend par extrait d'Eugenia jambos L. tout extrait obtenu à partir de la plante Eugenia jambos L.

Selon l'invention, l'extrait d'Eugenia jambos peut être un extrait de tout ou partie de la plante. Il peut notamment s'agir d'un extrait choisi parmi un extrait de feuilles, de graine, de fruit, de fleurs, ou un de leurs mélanges. Avantageusement, l'extrait peut être un extrait des feuilles de la plante.

L'extrait d'Eugenia jambos ou peut être un extrait alcoolique, aqueux, ou hydro-alcoolique, par exemple hydro-éthanolique. Par exemple, la composition peut comprendre tout ou partie des composés hydrophiles et hydrosolubles qui sont présents dans la plante, en particulier dans les feuilles de la plante, et qui en sont extraits au moyen d'un solvant choisi parmi l'eau, ou un alcool ou un mélange de plusieurs alcools, ou un mélange d'eau et d'au moins un alcool. La composition peut comprendre par exemple, des composés de types polyphénols, comme la myricetin-3-O-xylosyl(1->2) rhamnoside.

Le solvant d'extraction employé pour préparer l'extrait utilisé dans le cadre de la présente invention peut être choisi parmi les solvants connus de l'homme du métier, comme par exemple l'éthanol, le propylène glycol, le butylène glycol, le dipropylène glycol, le méthylpropanediol, le propane-1,3-diol, la glycérine, l'eau ou un mélange de ces solvants. Il peut s'agir par exemple d'un extrait obtenu à partir de feuilles, notamment extraites par un mélange hydroéthanolique. Alternativement, l'extrait d'Eugenia jambos peut-être obtenu par extraction hydroalcoolique, de préférence dans un mélange eau/éthanol ayant un rapport massique eau/alcool compris entre 90/10 et 10/90, par exemple de l'ordre de 70/ 30.

L'extrait d'Eugenia jambos peut être obtenu par tout procédé connu de l'homme du métier. Par exemple, l'extrait d'Eugenia jambos peut être obtenu en broyant (ou non) une ou plusieurs parties de la plante, de préférence séchées et coupées au préalable, puis en mettant en contact le broyat avec un solvant tel que défini précédemment, préférentiellement un mélange eau/alcool ayant un rapport eau/alcool entre 90/10 et 10/90, par exemple un mélange eau/alcool ayant un rapport massique 70/30. L'extraction peut être conduite en mélangeant le broyat et le solvant et en soumettant ce mélange à une agitation de façon à optimiser les échanges entre le broyat et le solvant d'extraction. L'extraction peut être conduite à une température de 20 à 100°C. Par exemple, l'extraction peut être réalisée pendant une durée de l'ordre de 1/2 à 4 heures. La quantité de plante utilisée par rapport au solvant peut être par exemple de 0,5 à 20 % en poids par rapport au poids total de plantes et de solvant, par exemple 1,0 à 20,0%, ou 2,0 à 20,0%, ou 3,0 à 15,0%, ou 5,0 à 12,0%, par exemple environ 10,0%. Il est à noter que l'extrait employé dans la présente invention peut être purifié préalablement à sa mise en œuvre dans la composition. Cette purification peut par exemple être effectuée par extraction liquide-liquide, par précipitation ou par chromatographie préparative.

Selon un mode de réalisation, on utilise comme extrait d'Eugenia jambos, l'extrait brut obtenu qui est issu de l'extraction décrite ci-dessus. Il peut donc s'agir du milieu tel qu'il est obtenu directement à l'issue de l'extraction par un des solvants précités, cet extrait brut étant éventuellement filtré préalablement avant son emploi dans la composition de l'invention. Dans ce mode de réalisation, l'extrait employé se présente sous la forme d'une dispersion ou d'une solution dans un milieu liquide incluant le solvant précité.

Selon un autre mode de réalisation, on utilise un extrait séché obtenu en soumettant l'extrait brut précité, de préférence filtré, à une étape ultérieure de séchage, typiquement par atomisation ou par lyophilisation, ou par séchage en étuve, selon des techniques connues, permettant de préserver l'intégrité des composés présents dans l'extrait. L'extrait obtenu selon ce mode de mise en œuvre se présente généralement sous la forme d'une poudre qui peut être employée telle qu'elle ou reprise dans un solvant ou un dispersant pour formuler la composition de la présente invention.

Un extrait selon l'invention est susceptible d'être préparé selon le procédé comprenant les étapes suivantes :
- Mise en contact de tout ou partie de la plante avec un solvant de type hydroalcoolique,
- Concentration,
- Filtration,
- Eventuellement, resolubilisation dans un solvant approprié ou séchage jusqu'à obtention d'une poudre.

Le procédé utilisé permet ainsi d'obtenir un extrait final avec une concentration importante en métabolites d'intérêt.

Les « métabolites », au sens de la présente invention, sont des composés ou molécules produits par des cellules d'une plante. Il peut s'agir de métabolites primaires, par exemples des sucres, protéines/acides aminés et/ou des lipides. Alternativement ou complémentairement, il peut s'agir de métabolites secondaires comme par exemple des polyphénols, des terpènes et/ou d'alcaloïdes.

Un deuxième objet de l'invention se rapporte à une composition cosmétique ou dermatologique comprenant au moins un extrait d'Eugenia jambos L. et un véhicule cosmétiquement et/ou dermatologiquement acceptable, ladite composition comprenant de 0,00001 à 5 % en poids dudit extrait par rapport au poids total de la composition. Ladite composition peut comprendre par exemple de 0,0001 à 5,0 % en poids, ou de 0,001 à 5,0 % en poids, ou de 0,01 à 5,0 % en poids ou de 0,1 à 5,0 % en poids, ou de 1,0 à 5,0 % en poids, ou de 1,0 à 4,0 %, ou de 2,0 à 4,0 %, par exemple 0,5% à 1,0% en poids dudit extrait d'Eugenia jambos L. par rapport au poids total de la composition.

On entend par « composition cosmétique », dans la présente invention, toute composition à visée cosmétique, c'est à dire esthétique, pouvant être mise en contact avec les parties superficielles du corps humain, par exemple l'épiderme, et les systèmes pileux et capillaires. Avantageusement, une composition cosmétique permet, exclusivement ou principalement, de les protéger, parfumer, maintenir en bon état, modifier leur aspect ou en corriger les défauts superficiels.

On entend par « composition dermatologique », dans la présente invention, toute composition à visée dermatologique, c'est à dire une composition pouvant être mise en contact avec les parties superficielles du corps humain, pour un traitement de la peau, des muqueuses, et des phanères, comme les ongles, les cheveux, ou les poils. Une telle composition peut comprendre des actifs additionnels autres que l'extrait d'Eugenia jambos L., susceptibles d'être qualifiés de dermatologiques ou thérapeutiques, ce qui explique la qualité de « dermatologique » de la composition.

Par « véhicule cosmétiquement ou dermatologiquement acceptable », on entend un véhicule adapté pour une utilisation en contact avec des cellules humaines cutanées, en particulier les cellules de l'épiderme, sans toxicité, irritation, réponse allergique indue et similaire, et proportionné à un rapport avantage/risque raisonnable.

Le véhicule cosmétiquement acceptable peut être choisi parmi l'eau, l'allantoïne, la glycérine, le méthylpropanediol ; cette liste n'étant pas limitative.

La composition cosmétique ou dermatologique de l'invention peut être obtenue par tout procédé approprié connu de l'homme du métier pour la fabrication d'une composition cosmétique. Il peut s'agir, par exemple d'un simple mélange. Il peut s'agir alternativement, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), ce procédé étant classiquement utilisé par l'homme de l'art pour obtenir des émulsions huile dans eau dont les gouttelettes dispersées sont particulièrement fines, par exemple avec un diamètre de 0,1 à 1 µm.

La composition cosmétique ou dermatologique de la présente invention peut se trouver sous toute forme appropriée pour une application cosmétique ou dermatologique. Avantageusement, la composition peut être une composition à usage topique.

Il peut s'agir par exemple d'une composition sous une forme choisie dans le groupe comprenant une émulsion huile dans eau ou eau dans huile ou un mélange de ces émulsions.

Selon l'invention, la composition cosmétique ou dermatologique peut par exemple se trouver sous une forme choisie dans le groupe comprenant un gel aqueux ou hydroalcoolique, une crème aqueuse ou hydroalcoolique et une lotion aqueuse ou hydroalcoolique. Ces formulations utilisables pour la mise en œuvre de la présente invention sont connues dans l'état de la technique par les formulateurs. Dans ces exemples de composition, il peut par exemple suffire d'ajouter l'extrait d'Eugenia jambos L. de la présente invention à une composition cosmétique ou dermatologique pour obtenir une composition conforme à la présente invention.

Selon l'invention, la composition peut être sous une forme choisie parmi un onguent, une crème, une huile, un lait, une pommade, une poudre, un tampon imbibé, une solution, un gel, un sérum, un baume, un beurre, une lotion, une suspension, un savon ou une émulsion.

L'extrait de la présente invention peut être utilisé dans une composition cosmétique ou dermatologique seul ou en combinaison avec d'autres substances ou ingrédients actifs ou inactifs cosmétiquement ou dermatologiquement. Les substances ou ingrédients inactifs sont ceux qui n'agissent pas cosmétiquement ou dermatologiquement. Il s'agit des éléments de la composition permettant notamment d'accompagner l'extrait, de constituer une formulation particulière, de conserver l'extrait actif dans le temps, cette liste n'étant pas limitative. Il peut s'agir en d'autres termes de tout produit de base pouvant se trouver dans les compositions cosmétiques ou dermatologiques classiques. Par opposition, les substances ou ingrédients actifs sont ceux qui, dans l'application cosmétique ou dermatologique visée, ont une action esthétique et/ou médicale.

Ainsi, l'extrait d'Eugenia jambos L. de la présente invention, peut être la seule substance ou ingrédient actif d'une composition, ou il peut être associé à d'autres substances ou ingrédients actifs d'une composition cosmétique ou dermatologique. Ainsi, l'extrait peut être utilisé en association avec tout ingrédient actif connu de l'homme du métier, apportant la même efficacité ou une efficacité complémentaire dans le domaine de l'anti-âge.

Un troisième objet de la présente invention se rapporte à un dispositif se présentant sous une forme choisie parmi un pot, un flacon-pompe, une lingette, un masque, un dispositif transdermique, un patch, un spray, ledit dispositif comprenant une composition telle que définie précédemment.

Un autre objet de l'invention se rapporte à un procédé cosmétique ou dermatologique non-thérapeutique comprenant l'application sur la peau ou les muqueuses d'une composition cosmétique selon. Le procédé de soin cosmétique ou dermatologique de l'invention permettant d'apporter notamment un effet notamment un effet anti-âge, par exemple au moins un effet choisi parmi l'amélioration de la régénération de la peau, la diminution de relâchement/perte de fermeté/perte d'élasticité, du lissage, l'action d'adoucir la peau, et l'amélioration de l'aspect général de la peau, notamment de l'éclat de la peau.

Dans le cadre des procédés cosmétiques selon l'invention, ou de l'utilisation selon l'invention, l'utilisation s'entend d'une utilisation non-thérapeutique, par exemple pour le traitement des peaux normales, c'est-à-dire des peaux ne présentant pas un état pathologique, à l'exclusion de toute utilisation thérapeutique. Ainsi, toute utilisation cosmétique et tout procédé cosmétique selon l'invention sont respectivement des utilisations cosmétiques non-thérapeutiques et procédés cosmétiques non-thérapeutiques.

D'autres avantages pourront encore apparaître à l'homme du métier à la lecture des exemples ci-dessous, donnés à titre illustratif.

### EXEMPLES OU MODES DE REALISATION

### Exemple 1 : Préparation d'extrait d'Eugenia jambos L.

Un extrait selon l'invention est préparé selon le procédé comprenant les étapes suivantes :
a. mise en contact de feuilles d'Eugenia jambos L. avec un solvant hydroéthanolique, c'est-à-dire avec un mélange eau/ éthanol ayant un rapport massique 70/30. La quantité de plante utilisée par rapport au solvant est de 10% en poids par rapport au poids total de plantes et de solvant,
b. extraction, pendant une durée de l'ordre de 45 min à 50°C,
c. filtration, pour la séparation du résidu sec et du jus d'extraction,
d. répétition des étapes a) et b) sur le résidu sec obtenu à l'étape c)
e. regroupement des 2 jus d'extraction issus des étapes c) et d)
f. concentration sous forme de poudre sans support.

### Exemple 2 : Effet de l'extrait d'Eugenia jambos L. obtenu selon l'Exemple 1 sur l'expression de médiateurs pro-inflammatoires

L'inflammation cutanée est un phénomène complexe mettant en jeu toutes les cellules de la peau et des cellules sanguines. L'une des premières étapes est l'expression de médiateurs pro-inflammatoires, notamment, l'interleukine 6 (IL-6) et le tumor necrosis factor alpha (TNFα) par les kératinocytes sous l'action d'un stress.

Les études ont été réalisées sur des kératinocytes épidermiques humains normaux, obtenus à partir de plasties abdominales, ensemencés en monocouche dans les conditions de culture adéquates, à savoir dans un milieu KSFM (Keratinocytes serum free médium, Invitrogen) à une température de 37°C, sous atmosphère à 5% de CO2 et saturée en humidité. Les cellules ont été traitées avec l'extrait d'Eugenia jambos L.(à 5.10⁻⁴% ou 5.10⁻⁵ %).

Après 24h de traitement avec les extraits, les cellules sont soumises aux radiations du simulateur solaire à raison de 760W/m² (Suntest CPS+). Puis 24 h après irradiation, les surnageants sont récoltés et les expressions de l'IL6 et du TNFα sont mesurées grâce à un test ELISA commercialisé par Bender MedSystem (BMS213INSTCE pour IL6 et BMS223INSTCE pour le TNFα). Ces résultats sont analysés au regard de la viabilité résiduelle des cellules (dosage d'activité mitochondriale au MTT).

Les données du tableau I (Expression de l'IL6 et du TNFα après irradiation des kératinocytes) ci-dessous résument les résultats obtenus.

**[Tableau 1]**

| | Plaque témoin non irradiée | | Plaque irradiée (UV) | | | |
|---|---|---|---|---|---|---|
| | [IL-6] en pg/ml | [TNFα] en pg/ml | [IL-6] en pg/ml | % IL6 | [TNFα] en pg/ml | % TNFα |
| Témoin milieu de culture | 69,0 | 507,8 | 137,7 | 100 | 743,2 | 100 |
| Eugenia 5.10⁻⁵% | 39,5 | 304,8 | 75,6 | 55 | 527,9 | 71 |
| Eugenia 5.10⁻⁴% | 35,1 | 33,0 | 68,7 | 50 | 257,9 | 35 |

Cet exemple montre que l'adjonction de l'extrait d'Eugénia jambos L. dans le milieu de culture protège les cellules suite au stress UV puisque le traitement permet de limiter l'expression des messagers pro-inflammatoires jusqu'à 50% pour l'IL6 et jusqu'à 65% pour le TNFα à 5.10⁻⁵% d'extraits d'Eugénia jambos L.

### Exemple 3 : Effet d'extrait d'Eugénia jambos L. obtenu selon l'Exemple 1 sur l'activité de la transglutaminase

Les études ont été réalisées sur des kératinocytes épidermiques humains normaux, obtenus à partir de plasties abdominales, ensemencés en monocouche dans les conditions de culture adéquates, à savoir dans un milieu kératinocyte-SFM, à une température de 37°C, sous atmosphère à 5% de CO2 et saturée en humidité. Les cellules ont été traitées avec l'extrait d'Eugénia Jambos L. pendant 96h à 3.10⁻⁴%. Puis l'activité de la transglutaminase est déterminée en mesurant l'incorporation de putrescine tritiée 2 µCi/ml final) par liaison covalente à la caséine, la caséine étant ensuite précipitée, récupérée sur des filtres. Enfin un comptage des filtres secs en scintillation liquide a été réalisé.

Les données du [Tableau 2] ci-dessous résument les résultats obtenus.

**[Tableau 2]**

| Traitement | % d'activité de la transglutaminase |
|---|---|
| Non traité | 100% |
| Extrait d'Eugénia Jambos L. à 3.10⁻⁴% | 124% |

Le traitement avec l'extrait d'Eugenia jambos L. permet de stimuler l'activité de la transglutaminase jusqu'à 24% à 3.10⁻⁴% chez les kératinocytes. Ces résultats montrent que l'extrait d'Eugenia jambos L. stimule l'activité des transglutaminases chez les kératinocytes favorisant ainsi leur différenciation et ainsi renforce les propriétés protectrices de l'épiderme.

### Exemple 4 : Formulation de l'extrait d'Eugenia jambos L. dans un soin de type sérum

**[Tableau 3]**

| Description | Quantité (%) |
|---|---|
| EAU PURIFIEE | qsp 100 |
| EAU DE BLEUET BIO | 1,1 |
| MANGIFERINE | 2 |
| GLYCERINE 100% VEG | 3 |
| GOMME XANTHANE | 0,1 |
| GOMME TARA | 0,2 |
| ALCOOL C16/C18 | 1 |
| ACETATE DE TOCOPHEROL | 0,1 |
| HUILE DE SESAME BIO | 1,1 |
| VITAMINE A | 0,1 |
| VITAMINE F | 0,3 |
| AMIDON DE MAIS | 1 |
| ALCOOL AGRICOLE 96% VOL | 6 |
| EXTRAIT EUGENIA JAM BOS | 1 |

### Exemple 5 : Formulation de l'extrait d'Eugenia jambos L. dans un soin de jour

**[Tableau 4]**

| Description | Qty% |
|---|---|
| EAU PURIFIEE | qsp 100 |
| MANGIFERINE | 2 |
| EAU DE BLEUET BIO SB YR | 1,1 |
| GLYCERINE 100% VEG | 5 |
| ACIDE SORBIQUE | 0,1 |
| METHYLPROPANE DIOL | 4 |
| GOMME XANTHANE standard | 0,15 |
| GOMME TARA | 0,5 |
| CIRES | 2 |
| METHYL GLUCOSE STEARATE | 2 |
| ACETATE DE TOCOPHEROL | 0,1 |
| LECITHINE DE SOJA | 0,2 |
| HUILE DE SESAME BIO DESODORISEE°° | 2 |
| HUILE DE COLZA RAFFINEE | 3 |
| HUILE DE SOJA IP°° | 3 |
| EXTRAIT EUGENIA JAMBOS | 1 |
| HYDROXYDE DE SODIUM | 0,05 |

### Exemple 6 : Formulation de l'extrait d'Eugenia jambos L. dans un soin de nuit

**[Tableau 5]**

| Description | Quantité% |
|---|---|
| EAU PURIFIEE | qsp 100 |
| GOMME XANTHANE | 0,15 |
| BUTYLENE GLYCOL | 3 |
| GOMME TARA | 0,5 |
| GLYCERINE 100% VEG | 6 |
| EAU DE BLEUET BIO | 1,1 |
| ACIDE SORBIQUE | 0,1 |
| ALCOOL C18-C22 | 3 |
| BEURRE DE KARITE | 2 |
| HUILE DE COPRAH | 2 |
| ACETATE DE TOCOPHEROL | 0,1 |
| LECITHINE DE SOJA D 300 | 0,2 |
| HUILE DE SOJA | 4 |
| VITAMINE A | 0,1 |
| VITAMINE F | 0,3 |
| EXTRAIT EUGENIA JAMBOS | 1 |

### Listes des références

1. Corine Djadjo Djipa et al. : "Antimicrobial activity of bark extracts of Syzygium jambos". J Ethnopharmacol. 2000 Jul;71(1-2):307-13.
2. P. Padma Rao : « Standardisation of Homoeopathic drug - Syzygium Jambos (L.) Alston ». New Delhi: Indian Journal of Research in Homoeopathy Vol. 5, No. 3, July - September, 2011.
3. Gavillán-Suárez et al. : « Chemical profile and in vivo hypoglycemic ». BMC Complement Altern Med. 2015 Jul 22;15:244.
4. K. Slowing, E. C. (1992). Anti-inflammatory activity of leaf extracts of Eugenia jambos. J Ethnopharmacol. 1994 Jun;43(1):9-11.
5. Avila-Peña D. et al.: " Antinociceptive activity of Syzygium jambos leaves extract on rats". J Ethnopharmacol. 2007 Jun 13;112(2):380-5.

## Revendications

1. Utilisation cosmétique d'un extrait d'Eugenia jambos L. pour une action anti-âge de la peau et/ou de ses annexes et/ou des muqueuses.

2. Utilisation cosmétique selon la revendication 1, dans laquelle ladite action anti-âge est au moins une action choisie parmi l'action de protéger, réparer la peau, préserver l'aspect, améliorer l'aspect, préserver les qualités biomécaniques, préserver et/ou renforcer la fonction barrière naturelle, de la peau et/ou de ses annexes et/ou des muqueuses.

3. Utilisation cosmétique selon la revendication 1 ou 2, dans laquelle l'action anti-âge est un effet de lissage, et/ou d'un effet d'amélioration de la douceur et/ou de l'homogénéité et/ou de l'éclat de la surface de la peau.

4. Utilisation cosmétique selon l'une quelconque des revendications précédentes, dans laquelle ledit extrait est choisi parmi un extrait de feuilles, de graine, de fruit, de fleurs, ou un de leurs mélanges.

5. Utilisation cosmétique selon la revendication 4, dans laquelle ledit extrait est un extrait de feuilles.

6. Utilisation cosmétique selon l'une quelconque des revendications précédentes, dans laquelle ledit extrait est choisi parmi un extrait alcoolique, aqueux, ou hydro-alcoolique.

7. Utilisation cosmétique selon la revendication 6, dans laquelle ledit extrait hydro-alcoolique est un extrait hydroéthanolique.

8. Utilisation cosmétique selon la revendication 7, dans laquelle ledit extrait hydro-alcoolique est un extrait hydroéthanolique de feuilles.

9. Composition cosmétique ou dermatologique comprenant au moins un extrait d'Eugenia jambos L. et un véhicule cosmétiquement et/ou dermatologiquement acceptable, ladite composition comprenant de 0,00001 à 5 % en poids dudit extrait par rapport au poids total de la composition.

10. Composition cosmétique ou dermatologique selon la revendication 9, ladite composition étant sous une forme choisie parmi un onguent, une crème, une huile, un lait, une pommade, une poudre, un tampon imbibé, une solution, un gel, un sérum, un baume, un beurre, une lotion, une suspension, un savon ou une émulsion.

11. Dispositif se présentant sous une forme choisie parmi un pot, un flacon-pompe, une lingette, un masque, un dispositif transdermique, un patch et un spray, ledit dispositif comprenant une composition selon l'une quelconque des revendications 9 ou 10.

12. Procédé cosmétique non-thérapeutique comprenant l'application sur la peau ou les muqueuses d'une composition cosmétique selon l'une quelconque des revendications 9 ou 10.
